# EUROPÄISCHE PATENTSCHRIFT

(11) **EP 1 491 222 B1**
(45) Veröffentlichungstag und Bekanntmachung des Hinweises auf die Patenterteilung: **20.04.2011**
(21) Anmeldenummer: 04012189.9
(22) Anmeldetag: 22.05.2004
(51) Int. Cl.: A61M 1/16

(54) **Vorrichtung zur extrakorporalen Blutbehandlung mit einer Einrichtung zur Überprüfung eines Sterilfilters und Verfahren zum Überprüfen eines Sterilfilters einer extrakorporalen Blutbehandlungsvorrichtung**
Device for extracorporal blood treatment with equipment for checking a sterile filter and associated method
Dispositif de traitement extracorporel du sang équipé pour tester un filtre stérile et méthode associée

(30) Priorität: 25.06.2003 DE 10328435
(43) Veröffentlichungstag der Anmeldung: 29.12.2004
(73) Patentinhaber: Fresenius Medical Care Deutschland GmbH, 61352 Bad Homburg v.d.H. (DE)
(72) Erfinder: Stahl, Thomas, 97839 Esselbach (DE)
(74) Vertreter: Oppermann, Frank

(56) Entgegenhaltungen:
- EP-A- 0 930 080
- EP-A- 0 974 371
- EP-A- 1 170 023

## Beschreibung

Die Erfindung bezieht sich auf eine Vorrichtung zur extrakorporalen Blutbehandlung mit einer Einrichtung zur Überprüfung der Bestückung der Blutbehandlungsvorrichtung mit einem im Dialysierflüssigkeitsweg anzuordnenden Sterilfilter oder zur Überprüfung der richtigen Größe eines im Dialysierflüssigkeitsweg angeordneten Sterilfilters. Darüber hinaus betrifft die Erfindung ein Verfahren zum Überprüfen der Bestückung einer Blutbehandlungsvorrichtung mit einem im Dialysierflüssigkeitsweg anzuordnenden Sterilfilter oder zum Überprüfen der richtigen Größe eines im Dialysierflüssigkeitsweg angeordneten Sterilfilters.

Es ist bekannt, die Dialysierflüssigkeit on-line aus frischem Wasser und einem oder mehreren Konzentraten herzustellen. Das Frischwasser weist in der Regel keine Keime auf und die Konzentrate sind üblicherweise steril, es kann jedoch nicht sichergestellt werden, dass die on-line hergestellte Dialysierflüssigkeit immer den sehr hohen Anforderungen an die Sterilität entspricht, die bei extrakorporalen Blutbehandlungen gestellt werden.

Bei der Hämodiafiltration ist es bekannt, die Substitutionsflüssigkeit on-line aus der Dialysierflüssigkeit herzustellen. Insbesondere die Substitutionsflüssigkeit hat den hohen Anforderungen an die Sterilität zu genügen.

Um die Sterilität der Dialysier- und Substitutionsflüssigkeit sicherzustellen, finden in den bekannten Blutbehandlungsvorrichtungen Sterilfilter Verwendung, die nach ein- oder mehrmaligem Gebrauch ausgetauscht werden können. Die EP 0 930 080 A1 beschreibt eine Blutbehandlungsvorrichtung, die über einen ersten Sterilfilter für die Herstellung einer sterilen Dialysierflüssigkeit aus Frischwasser und einem Dialysierflüssigkeitskonzentrat und einen zweiten Sterilfilter zur Herstellung einer sterilen Substitutionsflüssigkeit aus der Dialysierflüssigkeit verfügt. Beide Sterilfilter sind im Dialyiserflüssigkeitsweg stromauf des Dialysators angeordnet und werden von der Dialysierflüssigkeit durchspült.

In der Praxis ist sicherzustellen, dass ein für eine Blutbehandlungsvorrichtung vorgesehener Sterilfilter auch installiert ist. Ansonsten besteht die Gefahr, dass die Dialysierflüssigkeit und/oder Substitutionsflüssigkeit nicht steril ist. Die automatische Erkennung, ob die Vorrichtung mit einem Filter bestückt ist, macht im Allgemeinen einen zusätzlichen apparativen Aufwand erforderlich.

Der Erfindung liegt daher die Aufgabe zugrunde, eine Vorrichtung zur extrakorporalen Blutbehandlung zu schaffen, die mit einem relativ geringen zusätzlichen apparativen Aufwand die Überprüfung der Bestückung der Blutbehandlungsvorrichtung mit einem im Dialysierflüssigkeitsweg anzuordnenden Sterilfilter oder die Überprüfung der richtigen Größe eines im Dialysierflüssigkeitsweg angeordneten Sterilfilters erlaubt. Eine weitere Aufgabe der Erfindung liegt darin, ein Verfahren zum Überprüfen der Bestückung einer Blutbehandlungsvorrichtung mit einem im Dialysierflüssigkeitsweg anzuordnenden Sterilfilter oder zum Überprüfen der richtigen Größe eines im Dialysierflüssigkeitsweg angeordneten Sterilfilters anzugeben, das einen nur verhältnismäßig geringen apparativen Aufwand erfordert.

Die Lösung dieser Aufgaben erfolgt erfindungsgemäß mit den in den Patentansprüchen 1 und 10 angegebenen Merkmalen. Vorteilhafte Ausführungen der Erfindung sind Gegenstand der Unteransprüche.

Die Überprüfung des Sterilfilters beruht gemäß der Erfindung auf der Bestimmung des Zeitintervalls zwischen einer im Dialysierflüssigkeitsweg stromauf des Sterilfilters initiierten Veränderung einer physikalischen und/oder chemischen Eigenschaft der Dialysierflüssigkeit und der Detektion der Veränderung der Eigenschaft der Dialysierflüssigkeit im Dialysierflüssigkeitsweg stromab des Sterilfilters. Aus der Länge des ermittelten Zeitintervalls kann darauf geschlossen werden, ob die Blutbehandlungsvorrichtung mit einem Sterilfilter bestückt ist. Hierzu wird die Länge des ermittelten Zeitintervalls mit einem oder mehreren vorgegebenen Referenzwerten verglichen, die für einen bzw. verschiedene Sterilfiltern unterschiedlicher Ausbildung repräsentativ sind. Anstelle eines Zeitintervalls kann aber auch eine mit dem Zeitintervall korrelierende Größe, beispielsweise die Bilanzkammertakte ausgewertet werden, wenn die Blutbehandlungsvorrichtung über eine Bilanziereinrichtung auf Bilanzkammerbasis verfügt.

Wenn die Blutbehandlungsvorrichtung nicht mit einem Sterilfilter bestückt ist, d.h. die Anschlussklemmen des Sterilfilters mit einer Schlauchleitung überbrückt sind, benötigt eine impulsartige Veränderung der physikalischen und/oder chemischen Eigenschaften der Dialysierflüssigkeit, die stromauf des Sterilfilters initiiert wird, eine geringere Laufzeit, um stromab des Sterilfilters detektiert werden zu können, als wenn die Blutbehandlungsvorrichtung mit einem Sterilfilter bestückt ist. Dies ist auf das unterschiedliche Volumen an Dialysierflüssigkeit in dem vorgegebenen Abschnitt des Dialysierflüssigkeitsweges ohne oder mit Sterilfilter zurückzuführen.

Der besondere Vorteil der erfindungsgemäßen Vorrichtung bzw. des erfindungsgemäßen Verfahrens liegt darin, dass eine zusätzliche Sensorik an dem Sterilfilter selbst nicht erforderlich ist. Dadurch wird der apparative Aufwand verringert.

Die Überprüfung des Sterilfilters kann vor der eigentlichen Dialysebehandlung erfolgen. Die Behandlung beginnt damit erst dann, wenn sichergestellt ist, dass die Blutbehandlungsvorrichtung mit dem Sterilfilter bestückt ist.

Für die Überprüfung des Sterilfilters ist unerheblich, ob nur die erste, die zweite oder beide Kammern von der Dialysierflüssigkeit durchströmt werden, deren chemische und/oder physikalische Eigenschaft verändert wird. Daher kann der Sterilfilter auch dann überprüft werden, wenn er nur zum tangentialen Durchspülen in den Durchfluss geschaltet ist.

Die physikalische und/oder chemische Eigenschaft der Dialysierflüssigkeit kann jede Kenngröße sein, die in der Dialysierflüssigkeit nachweisbar ist. Vorzugsweise ist die physikalische und/oder chemische Eigenschaft die Konzentration einer bestimmten Substanz in der Dialysierflüssigkeit. Die physikalische und/oder chemische Kenngröße kann aber auch beispielsweise die Temperatur, die Dichte oder der Druck sein.

Die Messung der Konzentration einer bestimmten Substanz in der Dialysierflüssigkeit, beispielsweise Na, erfolgt vorzugsweise durch Messung der elektrischen Leitfähigkeit der Dialysierflüssigkeit. Vorteilhaft ist, dass mit der Leitfähigkeitsmessung bereits von im Dialysierflüssigkeitsweg der Blutbehandlungsvorrichtungen vorgesehenen Leitfähigkeitssensoren Gebrauch gemacht werden kann.

Zur Überprüfung des Sterilfilters genügt es, wenn die physikalische und/oder chemische Eigenschaft der Dialysierflüssigkeit nicht dauerhaft, sondern nur kurzfristig verändert wird. Vorzugsweise wird die Eigenschaft der Dialysierflüssigkeit nur für ein kurzes Zeitintervall sprunghaft verändert.

Bei einer besonders bevorzugten Ausgestaltung der Erfindung erfolgt die Veränderung der physikalischen und/oder chemischen Eigenschaft der Dialysierflüssigkeit dadurch, dass bei der Bereitstellung der Dialysierflüssigkeit das Mischungsverhältnis von Wasser und Konzentrat(en) vorzugsweise nur kurzzeitig verändert wird.

Neben der Überprüfung der Bestückung der Blutbehandlungsvorrichtung mit einem Sterilfilter erlaubt die erfindungsgemäße Vorrichtung bzw. das Verfahren auch die Überprüfung, ob die Blutbehandlungsvorrichtung mit einem Sterilfilter der richtigen Größe bestückt ist. Die Überprüfung der Größe des Sterilfilters erfolgt vorzugsweise durch Bestimmung des Volumens an Dialysierflüssigkeit, die durch den vorgegebenen Abschnitt des Dialyiserflüssigkeitsweges strömt, bis die Veränderung der physikalischen und/oder chemischen Eigenschaft der Dialysierflüssigkeit nachgewiesen werden kann. Dieses Volumen ist abhängig von dem Volumen des Sterilfilters, der in dem vorgegebenen Abschnitt des Dialysierflüssigkeitsweges von Dialysierflüssigkeit durchströmt wird. Ein Vergleich des ermittelten Volumens mit vorgegebenen Referenzwerten, die für die verschiedenen Typen von Sterilfiltern unterschiedlicher Größe repräsentativ sind, erlaubt die Erkennung des jeweiligen Sterilfilters.

Bei einer Blutbehandlungsvorrichtung, die über eine Bilanziereinrichtung zum Bilanzieren von frischer gegen verbrauchte Dialysierflüssigkeit verfügt, die mindestens eine Bilanzkammer aufweist, in der in aufeinanderfolgenden Bilanzkammertakten jeweils eine vorbestimmte Menge an Dialysierflüssigkeit gefördert wird, erfolgt die Bestimmung des Volumens an Dialysierflüssigkeit vorteilhafterweise aus der Anzahl der Bilanzkammerntakte und der vorbestimmten Menge an Dialysierflüssigkeit.

Im Folgenden wird ein Ausführungsbeispiel der Erfindung unter Bezugnahme auf die Zeichnungen näher erläutert.

Es zeigen:
- Figur 1: eine stark vereinfachte schematische Darstellung der wesentlichen Baugruppen einer Hämodialysevorrichtung mit einer Einrichtung zum Überprüfen des Sterilfilters, und
- Figur 2: die Leitfähigkeit der Dialysierflüssigkeit als Funktion der Zeit mit und ohne Sterilfilter.

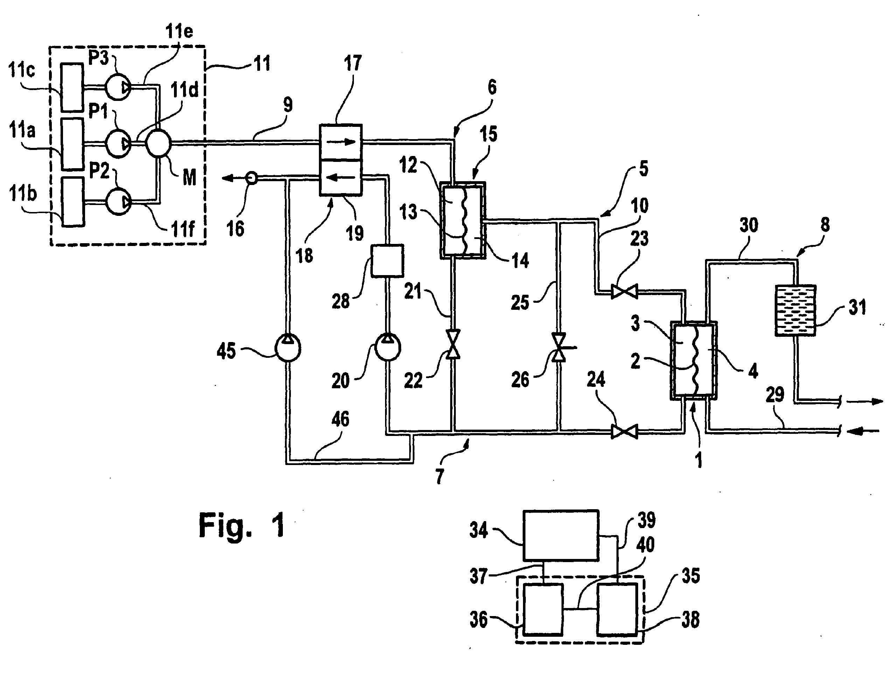

Figur 1 zeigt die wesentlichen Baugruppen einer Hämodialysevorrichtung in vereinfachter schematischer Darstellung. Die Dialysevorrichtung weist einen Dialysator 1 auf, der durch eine semipermeable Membran 2 in eine von Dialysierflüssigkeit durchflossene erste Kammer 3 und eine von Blut durchflossene zweite Kammer 4 geteilt ist. Die erste Kammer 3 ist in einen Dialysierflüssigkeitsweg 5 geschaltet, der eine Dialysierflüssigkeitszuführleitung 6 und eine Dialysierflüssigkeitsabführleitung 7 aufweist, während die zweite Kammer des Dialysators 1 in einen Blutweg 8 geschaltet ist.

Die Dialysierflüssigkeitszuführleitung 6 des Dialysierflüssigkeitsweges 5 weist einen ersten Leitungsabschnitt 9 und einen zweiten Leitungsabschnitt 10 auf. Der erste Leitungsabschnitt 9 verbindet eine Einrichtung 11 zur Bereitstellung von Dialysierflüssigkeit mit dem Einlaß einer ersten Kammer 12 eines durch eine Keime zurückhaltende Membran 13 in die erste Kammer 12 und eine zweite Kammer 14 unterteilten Sterilfilters 15. Der zweite Zuleitungsabschnitt 10 verbindet den Auslaß der zweiten Kammer 14 des Sterilfilters 15 mit dem Einlaß der ersten Kammer 3 des Dialysators. Der Auslaß der ersten Kammer 3 des Dialysators 1 ist über die Dialysierflüssigkeitsabführleitung 7 mit einem Auslauf 16 verbunden.

Die Einrichtung 11 zur Bereitstellung frischer Dialysierflüssigkeit weist eine Frischwasserquelle 11a sowie zwei Dialysierflüssigkeitskonzentratquellen 11b und 11c auf. Die Wasserquelle 11a ist über eine Wasserleitung 11d und die Konzentratquellen 11b, 11c sind über Konzentratleitungen 11e und 11f mit einem Mischpunkt M verbunden, von dem die Dialysierflüssigkeitzuführleitung 9 abgeht. In die Wasser- und Konzentratleitungen sind jeweils Proportionierungspumpen P1, P2 und P3 geschaltet, wobei mit den Flussraten der Pumpen das Mischungsverhältnis von Wasser und Konzentraten zum Mischen der Dialysierflüssigkeit eingestellt wird.

Zur Bilanzierung frischer gegen verbrauchte Dialysierflüssigkeit dient eine als Bilanzkammer ausgebildete Bilanziereinrichtung 18, die über eine erste und zweite Teilkammer 17, 19 verfügt. Die erste Teilkammer 17 ist in den ersten Leitungsabschnitt 9 der Dialysierflüssigkeitszuführleitung 6 geschaltet, während die zweite Teilkammer 19 in die Dialysierflüssigkeitsabführleitung 7 geschaltet ist. Stromauf der zweiten Teilkammer 19 ist in die Dialysierflüssigkeitsabführleitung eine Dialysierflüsigkeitspumpe 20 geschaltet. In der Praxis wird parallel zu der ersten Bilanzkammer 18 eine gegenphasige zweite Bilanzkammer verwendet, um einen nahezu kontinuierlichen Fluss zu ermöglichen. Auf die Darstellung der zweiten Bilanzkammer wurde aber der besseren Übersichtlichkeit halber verzichtet.

Von dem Auslaß der ersten Kammer 12 des Sterilfilters 15 führt eine erste Bypassleitung 21, in die ein erstes Bypassventil 22 geschaltet ist, zu der Dialysierflüssigkeitsabführleitung 7 stromauf der Dialysierflüssigkeitspumpe 20. Stromauf des Dialysators 1 ist in dem zweiten Leitungsabschnitt 10 der Dialysierflüssigkeitszuführleitung 6 ein erstes Absperrorgan 23 und stromab des Dialysators ist in der Dialysierflüssigkeitsabführleitung 7 ein zweites Absperrorgan 24 angeordnet. Eine zweite Bypassleitung 25, in die ein zweites Bypassventil 26 geschaltet ist, verbindet den zweiten Leitungsabschnitt 10 der Dialysierflüssigkeitszuführleitung 6 stromauf des ersten Absperrorgans 23 mit der Dialysierflüssigkeitsabführleitung 7 stromab des zweiten Absperrorgans 24.

In die Dialysierflüssigkeitsabführleitung 7 ist stromab der ersten und zweiten Bypassleitung 21, 25 und stromab der Dialysierflüssigkeitspumpe 20 eine Einrichtung 28 zur Messung der Leitfähigkeit der Dialysierflüssigkeit geschaltet. Stromab der ersten Bypassleitung 21 und stromauf der Dialysierflüssigkeitspumpe 20 zweigt von der Dialysierflüssigkeitsabführleitung 7 eine Ultrafiltrationsleitung 46 ab, die in die Dialysierflüssigkeitsabführleitung stromab der zweiten Teilkammer 19 mündet. In die Ultrafiltrationsleitung 46 ist eine Ultrafiltrationspumpe 45 geschaltet.

Der Blutweg 8 weist eine vom Patienten kommende Zuführleitung 29 auf, die an den Eingang der zweiten Kammer 4 des Dialysators 1 angeschlossen ist. Der Ausgang der zweiten Kammer 4 des Dialysators 1 führt über eine Blutabführleitung 30 zum Patienten, in die eine Tropfkammer 31 geschaltet ist.

Des weiteren verfügt die Dialysevorrichtung über eine zentrale Steuereinheit 34, die über nicht dargestellte Steuerleitungen die Dialysierflüssigkeits- und Ultrafiltrationspumpe 20, 45, die Proportionierungspumpen P1, P2, P3 und die Absperrorgane 22, 23, 24 und 26 sowie die Bilanziereinrichtung 18 ansteuert.

Während der Dialysebehandlung sind das erste und zweite Absperrorgan 23, 24 geöffnet und das erste und zweite Bypassventil 22, 26 geschlossen, so dass Dialysierflüssigkeit aus der Einrichtung 11 zur Bereitstellung der Diaylsierflüssigkeit über die erste Teilkammer 17 der Bilanziereinrichtung 18 und den Sterilfilter 15 in die erste Teilkammer 3 des Dialysators strömt. Aus der ersten Kammer des Dialysators strömt die Dialysierflüssigkeit dann über die zweite Teilkammer 19 der Bilanziereinrichtung 18 zu dem Auslauf 16. Dabei stellt der Sterilfilter 15 sicher, dass sterile Dialysierflüssigkeit den Dialysator 1 erreicht.

Zum Durchspülen des Sterilfilters 15 werden das erste und zweite Absperrorgan 23, 24 geschlossen und das erste Bypassventil 22 geöffnet, so dass die Dialysierflüssigkeit direkt in den Auslauf 16 abläuft. Die zweite Bypassleitung 25 bei geöffnetem zweiten Bypassventil 26 dient der Umgehung des Dialysators nach dem Spülbetrieb bei geschlossenem ersten und zweiten Absperrorgan 23, 24. Auch hier fließt die Dialysierflüssigkeit direkt in den Auslauf 16 ab.

Der Sterilfilter 15 ist zum einmaligen oder mehrmaligen Gebrauch bestimmt und kann unter Kurzschluss der Leitungen ausgetauscht werden. Eine Einrichtung 35 zur Überprüfung des Sterilfilters stellt sicher, dass die Dialysevorrichtung zu Beginn der eigentlichen Dialysebehandlung mit einem Sterilfilter bestückt ist. Die Einrichtung 35 zur Überprüfung des Sterilfilters 15 weist eine Recheneinheit 36 auf, die über eine Datenleitung 37 mit der zentralen Steuereinheit 34 und über eine Datenleitung 40 mit einer Alarmeinheit 38 verbunden ist, die einen akustischen und/oder optischen Alarm ergibt. Die Alarmeinheit 38 ist über eine Datenleitung 39 wiederum mit der Steuereinheit 34 verbunden, die im Falle eines Alarms die Routine zur Vorbereitung der Dialysebehandlung zur Bestückung der Dialysemaschine mit einem Sterilfilter unterbricht.

Während der Routine zur Vorbereitung der Dialysebehandlung arbeitet die Einrichtung 35 zur Überprüfung des Sterilfilters wie folgt.

Zunächst wird der Dialysierflüssigkeitsweg 6 mit Frischwasser gespült, wobei nur die Proportionierungspumpe P1, nicht aber die Pumpen P2 und P3 betrieben werden. Anschließend wird eine der beiden Pumpen P2 und P3 zur Erzeugung eines Konzentratbolus kurzzeitig angesteuert, der als Leitfähigkeitsimpuls in der Dialysierflüssigkeit nachgewiesen werden kann. Dieser Leitfähigkeitsimpuls breitet sich über die Dialysierflüssigkeitszuführleitung 6, die den Sterilfilter 15 einschließt, den Dialysator 1 und die Dialysierflüssigkeitsabführleitung 7 aus, bis er die Einrichtung 28 zur Messung der Leitfähigkeit der Dialysierflüssigkeit erreicht und detektiert wird.

Die Recheneinheit 36 weist Mittel zum Bestimmen des Zeitintervalls Δt zwischen dem ersten Zeitpunkt, zu dem der Leitfähigkeitsimpuls mit der Einrichtung 11 zur Bereitstellung der Dialysierflüssigkeit erzeugt wird, und dem zweiten Zeitpunkt auf, zu dem der Leitfähigkeitsimpuls mit der Einrichtung 28 zur Messung der Leitfähigkeit detektiert wird. Darüber hinaus weist die Recheneinheit 36 Mittel zum Vergleichen der Länge des Zeitintervalls Δt mit vorgegebenen Referenzwerten auf, die für die verwendeten Sterilfiter 15 repräsentativ sind. Der Zeitpunkt t, zu dem die Messung beginnt, kann durch beliebige Signale definiert werden, die mit dem Fortschreiten der Änderung der Eigenschaft, beispielweise dem Leitfähigkeitsimpuls, einhergehen. Zur Detektion des Leitfähigkeitsimpulses an einem bestimmten Punkt der Messstrecke zu dem Zeitpunkt des Beginns der Messung kann ein weiterer Leitfähigkeitssensor vorgesehen sein.

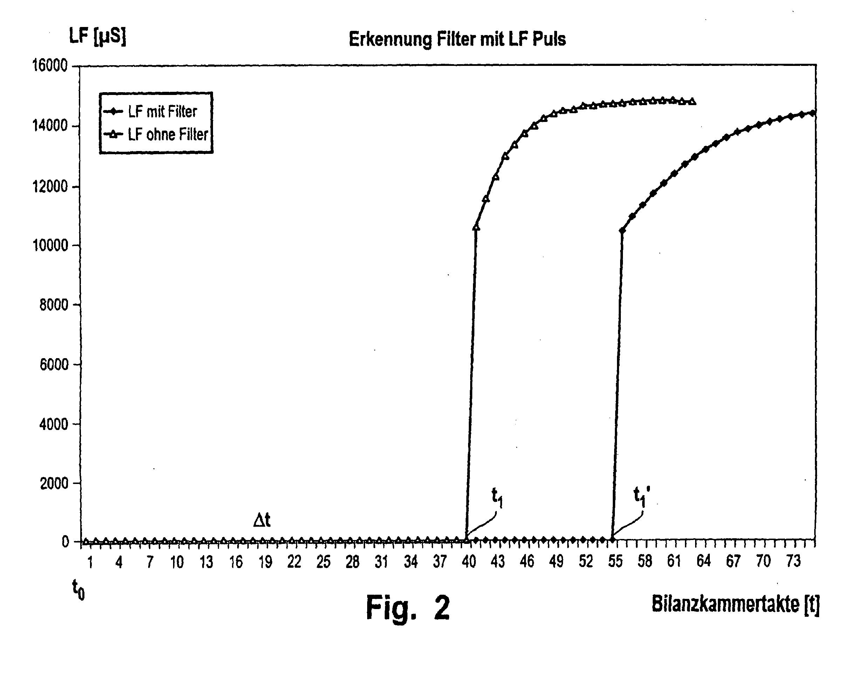

Figur 2 zeigt die Leitfähigkeit der Dialysierflüssigkeit als Funktion der Zeit, wobei die Zeitachse in Bilanzkammertakten angegeben ist. Da die Bilanziereinrichtung 18 in aufeinanderfolgenden Bilanzkammertakten jeweils eine vorbestimmte Menge an Dialysieflüssigkeit fördert, besteht zwischen dem Volumen der geförderten Dialysierflüssigkeit und der Anzahl der Bilanzkammertakte ein fest vorgegebener Zusammenhang. Im vorliegenden Beispiel ist ein Dialysierflüssigkeitsfluss von 500 ml/min eingestellt, wobei ein Bilanzkammertakt 3,3 s dauert. Eine Bilanzkammerfüllung umfasst im vorliegenden Ausführungsbeispiel 30 m/l. Damit entsprechen beispielsweise 15 Bilanzkammertakte einer Zeitverschiebung von 49,5 s und einem Volumenunterschied von 450 ml.

Figur 2 zeigt, dass der zum Zeitpunkt t = 0 initiierte Leitfähigkeitsimpuls zum Zeitpunkt t=t₁ nach einer Zeitverschiebung Δt von 132 s, was
40 Bilanzkammertakten entspricht, nachgewiesen werden kann, wenn die Dialysevorrichtung nicht mit einem Sterilfilter 15 bestückt ist. Wenn die Dialysevorrichtung mit einem Sterilfilter 15 bestückt ist, wird der Leitfähigkeitsimpuls erst zum Zeitpunkt t=t₁'nach 55 Bilanzkammertakten, d.h. 181,5 s nachgewiesen. Dabei erfolgt der Nachweis des Leitfähigkeitsimpulses durch die Detektion des sprunghaften Anstiegs der Leitfähigkeit.

In der Recheneinheit 36 wird die Anzahl der Bilanzkammertakte, die der Zeitverschiebung entspricht, mit einem vorgegebenen Referenzwert verglichen, der zwischen 40 und 55 Bilanzkammertakten, beispielsweise 50 Bilanzkammertakte, liegt. Für den Fall, dass die ermittelte Zeitverschiebung kleiner als der Referenzwert ist, gibt die Recheneinheit 36 ein Alarmsignal an die Alarmeinheit 38, die einen akustischen/oder optischen Alarm gibt und über die Steuereinheit die Routine zur Vorbereitung der Dialysebehandlung unterbricht.

Die Zeitverschiebung Δt ist abhängig von dem Volumen der Dialysierflüssigkeit, die durch den vorgegebenen Teil des Dialysierflüssigkeitsweges fließt, der den sterilen Filter einschließt. Ein Sterilfilter mit einem größeren Volumen verursacht folglich eine größere Zeitverschiebung als ein Sterilfilter mit einem kleineren Volumen.

Zur Erkennung eines Sterilfilters mit einem bestimmten Volumen vergleicht die Recheneinheit die Zeitverschiebung mit vorgegebenen Grenzwerten, die jeweils für einen Sterilfilter mit einem bestimmten Volumen charakteristisch sind. Ist die Zeitverschiebung größer als der jeweils charakteristische Grenzwert, wird darauf geschlossen, dass der jeweilige Sterilfilter eingesetzt ist.

Bei den beschriebenen Ausführungsbeispielen kann der Sterilfilter 15 bei einem Flüssigkeitsfluss sowohl durch den Dialysator 1 als auch durch die erste oder zweite Bypassleitung 21, 25 überprüft werden, da die Einrichtung 28 zur Messung der Leitfähigkeit stromab der ersten und zweiten Bypassleitung 21, 25 angeordnet ist. Die Einrichtung 28 kann aber auch im zweiten Abschnitt der Dialysierflüssigkeitzuführleitung 10 angeordnet sein. Dann ist eine Überprüfung des Sterilfilters allerdings nicht bei einem Flüssigkeitsfluss durch die erste Bypassleitung 21 möglich. Andererseits ist das vorgegebene Volumen, das den Sterilfilter einschließt bei einer derartigen Anordnung der Einrichtung 28 geringer, da ein Teil der Dialysierflüssigkeitszuführ- und abführleitung entfällt. Gleichzeitig ändern sich die zu verwendenden Referenzwerte. Insbesondere ist bei der Messung über die Bypassleitung 21 zu berücksichtigen, dass nur eine Kammer des Sterilfilters 15 durchflossen wird.

## Patentansprüche

1. Vorrichtung zur extrakorporalen Blutbehandlung mit
einem Dialysator (1), der durch eine semipermeable Membran (2) in eine erste und zweite Kammer geteilt ist, wobei die erste Kammer (3) in einem Dialysierflüssigkeitsweg (5) und die zweite Kammer (4) in einem Blutweg (8) angeordnet ist,
einem im Dialysierflüssigkeitsweg anzuordnenden oder angeordneten Sterilfilter (15), der durch eine Keime zurückhaltende Membran (13) in eine erste Kammer (12) und eine zweite Kammer (14) geteilt ist,
einer Einrichtung (11) zur Bereitstellung frischer Dialysierflüssigkeit und einem Auslauf (16) für verbrauchte Dialysierflüssigkeit,
einer Einrichtung (11) zur Veränderung einer chemischen und/oder physikalischen Eigenschaft der Dialysierflüssigkeit in dem Dialysierflüssigkeitsweg stromauf des im Dialysierflüssigkeitsweg anzuordnenden oder angeordneten Sterilfilters (15) und einer Einrichtung (28) zur Messung der Veränderung der chemischen und/oder physikalischen Eigenschaft der Dialysierflüssigkeit im Dialysierflüssigkeitsweg stromab des in dem Dialysierflüssigkeitsweg anzuordnenden oder angeordneten Sterilfilters,
einer Einrichtung (35) zur Überprüfung der Bestückung der Blutbehandlungsvorrichtung mit einem im Dialysierflüssigkeitsweg anzuordnenden Sterilfilter (15) oder zur Überprüfung der richtigen Größe eines im Dialysierflüssigkeitsweg angeordneten Sterilfilters (15),
**dadurch gekennzeichnet, dass** die Überprüfungseinrichtung (35) aufweist:
Mittel (36) zum Bestimmen des Zeitintervalls Δt zwischen einem ersten Zeitpunkt t₀, zu dem die physikalische und/oder chemische Eigenschaft der Dialysierflüssigkeit stromauf des Sterilfilters mit der Einrichtung (11) zum Verändern der Eigenschaft der Dialysierflüssigkeit verändert wird und einem zweiten Zeitpunkt t₁, zu dem die Veränderung der Eigenschaft der Dialysierflüssigkeit mit der Einrichtung (28) zur Messung der Veränderung der Dialysierflüssigkeit detektiert wird, und
Mitteln (36) zum Vergleichen der Länge des ermittelten Zeitintervalls oder einer mit dem ermittelten Zeitintervall korrelierenden Größe mit mindestens einem vorgegebenen Referenzwert.

2. Vorrichtung nach Anspruch 1, **dadurch gekennzeichnet, dass** die physikalische und/oder chemische Eigenschaft der Dialysierflüssigkeit die Konzentration einer bestimmten Substanz in der Dialysierflüssigkeit ist.

3. Vorrichtung nach Anspruch 2, **dadurch gekennzeichnet, dass** die Einrichtung zur Messung der Konzentration einer bestimmten Substanz in der Dialysierflüssigkeit eine Einrichtung (28) zur Messung der Leitfähigkeit der Dialysierflüssigkeit ist.

4. Vorrichtung nach einem der Ansprüche 1 bis 3, **dadurch gekennzeichnet, dass** die Einrichtung (11) zur Bereitstellung der Dialysierflüssigkeit eine Wasserquelle (11a) und mindestens eine Konzentratquelle (11b, 11c) und Mittel (M) zum Mischen von Wasser und Konzentrat(en) aufweist, wobei die Einrichtung zum Verändern der physikalischen und/oder chemischen Eigenschaft der Dialysierflüssigkeit eine das Mischungsverhältnis von Wasser und Konzentrat(en) verändernde Einrichtung ist.

5. Vorrichtung nach einem der Ansprüche 1 bis 4, **dadurch gekennzeichnet, dass** die Überprüfungseinrichtung (35) eine Einrichtung (36) zum Bestimmung des Volumens an Dialysierflüssigkeit aufweist, die zwischen dem ersten Zeitpunkt t₀, zu dem die physikalische und/oder chemische Eigenschaft der Dialysierflüssigkeit stromauf des Sterilfilters (15) mit der Einrichtung (11) zum Verändern der Eigenschaft der Dialysierflüssigkeit verändert wird, und dem zweiten Zeitpunkt t₁, zu dem die Veränderung der Eigenschaft der Dialysierflüssigkeit mit der Einrichtung (28) zur Messung der Veränderung der Eigenschaft der Dialysierflüssigkeit detektiert wird, durch einen vorgegebenen Abschnitt des Dialysierflüssigkeitsweges (5) strömt, der den Sterilfilter (15) einschließt.

6. Vorrichtung nach Anspruch 5, **dadurch gekennzeichnet, dass** die Überprüfungseinrichtung (35) Mittel (36) zum Vergleichen des ermittelten Volumens mit mindestens einem vorgegebenen Referenzwert aufweist.

7. Vorrichtung nach Anspruch 5 oder 6, **dadurch gekennzeichnet, dass** im Dialysierflüssigkeitsweg (5) eine Bilanzeinrichtung (18) zum Bilanzieren von frischer gegen verbrauchte Dialysierflüssigkeit angeordnet ist, die mindestens eine zwei Teilkammern unfassende Bilanzkammer (17, 19) aufweist, in der in aufeinanderfolgenden Bilanzkammertakten jeweils eine vorbestimmte Menge an Dialysierflüssigkeit gefördert wird, und die Mittel (36) zum Bestimmen des Volumens an Dialysierflüssigkeit derart ausgebildet sind, dass das Volumen an Dialysierflüssigkeit aus der Anzahl der Bilanzkammertakte und der vorbestimmten Menge an Dialysierflüssigkeit bestimmbar ist.

8. Vorrichtung nach einem der Ansprüche 1 bis 7, **dadurch gekennzeichnet, dass** der Dialysierflüssigkeitsweg (5) einen ersten Leitungsabschnitt (9) einer Dialysierflüssigkeitszuführleitung (6), der von der Einrichtung (11) zur Bereitstellung von Dialysierflüssigkeit abgeht und zu dem Einlaß der ersten Kammer (12) des Sterilfilters (15) führt, und einen zweiten Leitungsabschnitt (10) der Dialysierflüssigkeitszuführleitung (6) aufweist, der von der zweiten Kammer (14) des Sterilfilters (15) abgeht und zu dem Einlaß des Dialysators (1) führt, und
der Dialysierflüssigkeitsweg (5) eine Dialysierflüssigkeitsabführleitung (7) aufweist, die von dem Auslaß des Dialysators (1) abgeht und zu dem Auslauf (16) führt, wobei
die Einrichtung (28) zur Messung der physikalischen und/oder chemischen Eigenschaft der Dialysierflüssigkeit eine die physikalische und/oder chemische Eigenschaft der Dialysierflüssigkeit in der Dialysierflüssigkeitsabführleitung (7) messende Einrichtung ist.

9. Vorrichtung nach Anspruch 8, **dadurch gekennzeichnet, dass** der Dialysierflüssigkeitsweg (6) eine von einem Auslaß der ersten Kammer (12) des Sterilfilters (15) abgehende und zu der Dialysierflüssigkeitsabführleitung (7) führende Bypassleitung (21) aufweist, wobei
die Einrichtung (28) zur Messung der physikalischen und/oder chemischen Eigenschaft der Dialysierflüssigkeit eine die Eigenschaft der Dialysierflüssigkeit in der Dialysierflüssigkeitsabführleitung (7) stromab der Bypassleitung (21) messende Einrichtung ist.

10. Verfahren zum Überprüfen der Bestückung einer Vorrichtung zur extrakorporalen Blutbehandlung mit einem im Dialysierflüssigkeitsweg der Blutbehandlungsvorrichtung anzuordnenden Sterilfilter (15) oder zur Überprüfung der richtigen Größe eines im Dialysierflüssigkeitsweg angeordneten Sterilfilters (15) vor Beginn der extrakorporalen Blutbehandlung, mit folgenden Verfahrensschritten:
Verändern einer physikalischen und/oder chemischen Eigenschaft der Dialysierflüssigkeit im Dialysierflüssigkeitsweg stromauf des im Dialysierflüssigkeitsweg anzuordnenden oder angeordneten Sterilfilters (15) und Messen der Veränderung der Eigenschaft der Dialysierflüssigkeit im Dialysierflüssigkeitsweg stromab des im Dialysierflüssigkeitsweg anzuordnenden oder angeordneten Sterilfilters,
Bestimmen des Zeitintervalls Δt zwischen einem ersten Zeitpunkt t₀, zu dem die physikalische und/oder chemische Eigenschaft der Dialysierflüssigkeit stromauf des Sterilfilters verändert wird, und einem zweiten Zeitpunkt t₁, zu dem die Veränderung der Eigenschaft der Dialysierflüssigkeit stromab des Sterilfilters detektiert wird, und
Vergleichen des ermittelten Zeitintervalls Δt oder einer damit korrelierenden Größe mit mindestens einem vorgegebenen Referenzwert.

11. Verfahren nach Anspruch 10, **dadurch gekennzeichnet, dass** die physikalische und/oder chemische Eigenschaft der Dialysierflüssigkeit die Konzentration einer bestimmten Substanz in der Dialysierflüssigkeit ist.

12. Verfahren nach Anspruch 11, **dadurch gekennzeichnet, dass** zum Bestimmen der Konzentrationen einer bestimmten Substanz in der Dialysierflüssigkeit die Leitfähigkeit der Dialysierflüssigkeit gemessen wird.

13. Verfahren nach einem der Ansprüche 10 bis 12, **dadurch gekennzeichnet, dass** zur Bereitstellung von Dialysierflüssigkeit Wasser und mindestens ein Konzentrat miteinander gemischt werden, wobei zum Verändern der physikalischen und/oder chemischen Eigenschaft der Dialysierflüssigkeit das Mischungsverhältnis von Wasser und Konzentrat(en) verändert wird.

14. Verfahren nach einem der Ansprüche 10 bis 13, **dadurch gekennzeichnet, dass** das Volumen der Dialysierflüssigkeit bestimmt wird, die zwischen dem ersten Zeitpunkt t₀, zu dem die physikalische und/oder chemische Eigenschaft der Dialysierflüssigkeit stromauf des Sterilfilters verändert wird, und dem zweiten Zeitpunkt t₁, zu dem die Veränderung der Eigenschaft der Dialysierflüssigkeit detektiert wird, durch einen vorgegebenen Abschnitt des Dialysierflüssigkeitsweges strömt, der den Sterilfilter einschließt.

15. Verfahren nach Anspruch 14, **dadurch gekennzeichnet, dass** das ermittelte Volumen mit mindestens einem vorgegebenen Referenzwert verglichen wird.

16. Verfahren nach einem der Ansprüche 10 bis 15, **dadurch gekennzeichnet, dass** frische gegen verbrauchte Dialysierflüssigkeit in einer Bilanziereinrichtung bilanziert werden, die in aufeinanderfolgenden Bilanzkammertakten jeweils eine vorbestimmte Menge an Dialysierflüssigkeit fördert, wobei das Volumen an Dialysierflüssigkeit aus der Anzahl der Bilanzkammertakte und der vorbestimmten Menge an Dialysierflüssigkeit bestimmt wird.

## Claims

1. An apparatus for extracorporeal blood treatment with
a dialyser (1), which is divided by a semi-permeable membrane (2) into a first and second chamber, wherein the first chamber (3) is disposed in a dialysing fluid path (5) and the second chamber (4) is disposed in a blood path (8),
a sterile filter (15) disposed or to be disposed in the dialysing fluid path, said sterile filter being divided by a germ-restraining membrane (13) into a first chamber (12) and a second chamber (14),
a device (11) for preparing fresh dialysing fluid and a drain (16) for used dialysing fluid,
a device (11) for changing a chemical and/or physical property of the dialysing fluid in the dialysing fluid path upstream of the sterile filter (15) disposed or to be disposed in the dialysing fluid path (15) and
a device (28) for measuring the change in the chemical and/or physical property of the dialysing fluid in the dialysing fluid path downstream of the sterile filter disposed or to be disposed in the dialysing fluid path,
a device (35) for checking the installed equipment of the blood treatment apparatus with a sterile filter (15) to be disposed in the dialysing fluid path or for checking the correct size of a sterile filter (15) disposed in the dialysing fluid path,
**characterised in that** the checking device (35) comprises:
means (36) for determining time interval At between a first time t₀, at which the physical and/or chemical property of the dialysing fluid upstream of the sterile filter is changed with the device (11) for changing the property of the dialysing fluid, and a second time t₁, at which the change in the property of the dialysing fluid is detected with the device (28) for measuring the change in the dialysing fluid, and means (36) for comparing the length of the ascertained time interval or a variable correlating with the ascertained time interval with at least one predetermined reference value.

2. The device according to claim 1, **characterised in that** the physical and/or chemical property of the dialysing fluid is the concentration of a specific substance in the dialysing fluid.

3. The device according to claim 2, **characterised in that** the device for measuring the concentration of a specific substance in the dialysing fluid is a device (28) for measuring the conductivity of the dialysing fluid.

4. The device according to any one of claims 1 to 3, **characterised in that** the device (11) for preparing the dialysing fluid comprises a water source (11a) and at least one concentrate source (11b, 11c) and means (M) for mixing water and concentrate(s), wherein the device for changing the physical and/or chemical property of the dialysing fluid is a device which changes the mixing ratio of water and concentrate(s).

5. The device according to any one of claims 1 to 4, **characterised in that** the checking device (35) comprises a device (36) for determining the volume of dialysing fluid which flows through a predetermined segment of the dialysing fluid path (5) comprising the sterile filter (15) between first time t₀, at which the physical and/or chemical property of the dialysing fluid upstream of the sterile filter (15) is changed with the device (11) for changing the property of the dialysing fluid, and second time t₁, at which the change in the property of the dialysing fluid is detected with the device (28) for measuring the change in the property of the dialysing fluid.

6. The device according to claim 5, **characterised in that** the checking device (35) comprises means (36) for comparing the ascertained volume with at least one predetermined reference value.

7. The device according to claim 5 or 6, **characterised in that** a balancing arrangement (18) for balancing fresh dialysing fluid against used dialysing fluid is disposed in the dialysing fluid path (5), said balancing arrangement comprising at least one balancing chamber (17, 19), which comprises two sub-chambers and in which a predetermined quantity of dialysing fluid is conveyed each time in successive balancing chamber cycles, and the means (36) for determining the volume of dialysing fluid are designed in such a way that the volume of dialysing fluid can be determined from the number of balancing chamber cycles and the predetermined quantity of dialysing fluid.

8. The device according to any one of claims 1 to 7, **characterised in that** the dialysing fluid path (5) comprises a first line segment (9) of a dialysing fluid supply line (6), which leads away from the device (11) for preparing dialysing fluid and leads to the inlet of the first chamber (12) of the sterile filter (15), and a second line segment (10) of the dialysing fluid supply line (6), which leads away from the second chamber (14) of the sterile filter (15) and leads to the inlet of the dialyser (1), and
the dialysing fluid path (5) comprises a dialysing fluid discharge line (7), which leads away from the outlet of the dialyser (1) and leads to the drain (16), wherein
the device (28) for measuring the physical and/or chemical property of the dialysing fluid is a device which measures the physical and/or chemical property of the dialysing fluid in the dialysing fluid discharge line (7).

9. The device according to claim 8, **characterised in that** the dialysing fluid path (6) comprises a bypass line (21) leading away from an outlet of the first chamber (12) of the sterile filter (15) and leading to the dialysing fluid discharge line (7), wherein
the device (28) for measuring the physical and/or chemical property of the dialysing fluid is a device which measures the property of the dialysing fluid in the dialysing fluid discharge line (7) downstream of the bypass line (21).

10. A method for checking the installed equipment of an apparatus for extracorporeal blood treatment with a sterile filter (15) to be disposed in the dialysing fluid path of the blood treatment apparatus or for checking the correct size of a sterile filter (15) disposed in the dialysing fluid path before the start of the extracorporeal blood treatment, with the following process steps:
changing a physical and/or chemical property of the dialysing fluid in the dialysing fluid path upstream of the sterile filter (15) disposed or to be disposed in the dialysing fluid path and measuring the change in the property of the dialysing fluid in the dialysing fluid path downstream of the sterile filter disposed or to be disposed in the dialysing fluid path,
determination of time interval Δt between a first time t₀, at which the physical and/or chemical property of the dialysing fluid upstream of the sterile filter is changed, and a second time t₁, at which the change in the property of the dialysing fluid downstream of the sterile filter is detected, and
comparison of ascertained time interval At or a variable correlating therewith with at least one predetermined reference value.

11. The method according to claim 10, **characterised in that** the physical and/or chemical property of the dialysing fluid is the concentration of a specific substance in the dialysing fluid.

12. The method according to claim 11, **characterised in that** the conductivity of the dialysing fluid is measured in order to determine the concentrations of a specific substance in the dialysing fluid.

13. The method according to any one of claims 10 to 12, **characterised in that** water and at least one concentrate are mixed together in order to prepare the dialysing fluid, wherein the mixing ratio of water and concentrate(s) is changed in order to change the physical and/or chemical property of the dialysing fluid.

14. The method according to any one of claims 10 to 13, **characterised in that** the volume of the dialysing fluid is determined which flows through a predetermined segment of the dialysing fluid path comprising the sterile filter between first time t₀, at which the physical and/or chemical property of the dialysing fluid upstream of the sterile filter is changed, and second time t₁, at which the change in the property of the dialysing fluid is detected.

15. The method according to claim 14, **characterised in that** the ascertained volume is compared with at least one predetermined reference value.

16. The method according to any one of claims 10 to 15, **characterised in that** fresh dialysing fluid is balanced against used dialysing fluid in a balancing arrangement, which conveys a predetermined quantity of dialysing fluid each time in successive balancing chamber cycles, wherein the volume of dialysing fluid is determined from the number of balancing chamber cycles and the predetermined quantity of dialysing fluid.

## Revendications

1. Dispositif de traitement extracorporel du sang avec
■ un dialyseur (1), qui est divisé en une pre-mière et une seconde chambre par une membrane semi-perméable (2), dans lequel la première chambre (3) est disposée sur le chemin de la solution de dialyse (5) et la seconde chambre (4) sur le chemin du sang (8),
■ un filtre stérile (15) disposé ou à disposer sur le chemin de la solution de dialyse, qui est divisé en une première chambre (12) et une seconde chambre (14) par une membrane (13) retenant des germes,
■ un dispositif (11) pour la mise à disposition de solution de dialyse fraîche et une sortie (16) pour la solution de dialyse usée,
■ un dispositif (11) pour la modification d'une propriété chimique et/ou physique de la solu-tion de dialyse sur le chemin de la solution de dialyse en amont du filtre stérile (15) disposé ou à disposer sur le chemin de la solution de dialyse et
■ un dispositif (28) pour mesurer la modification de la propriété chimique et/ou physique de la solution de dialyse sur le chemin de la solu-tion de dialyse en aval du filtre stérile disposé ou à disposer sur le chemin de la solution de dialyse,
■ un dispositif (35) pour contrôler l'équipement du dispositif de traitement du sang, équipé d'un filtre stérile (15) à disposer sur le chemin de la solution de dialyse ou pour véri-fier la taille correcte d'un filtre stérile (15) disposé sur le chemin de la solution de dialyse,
**caractérisé en ce que** le dispositif de contrôle (35) présente :
■ des moyens (36) pour déterminer l'intervalle de temps Δt entre un premier moment t₀, où la pro-priété physique et/ou chimique de la solution de dialyse en amont du filtre stérile est modifiée avec le dispositif (11) pour modifier la propriété de la solution de dialyse et un second moment t₁, où la modification de la propriété de la solution de dialyse est détec-tée avec le dispositif (28) pour mesurer la modification de la solution de dialyse, et
■ des moyens (36) pour comparer la longueur de l'intervalle de temps déterminé ou une grandeur en corrélation avec l'intervalle de temps déter-miné à au moins une valeur de référence prescrite.

2. Dispositif selon la revendication 1, caracté-risé en ce que la propriété physique et/ou chimique de la solution de dialyse est la concentration d'une certaine substance dans la solution de dialyse.

3. Dispositif selon la revendication 2, caracté-risé en ce que le dispositif pour mesurer la concentration d'une certaine substance dans la solution de dialyse est un dispositif (28) pour mesurer la conductibilité de la solution de dialyse.

4. Dispositif selon l'une quelconque des revendi-cations 1 à 3, **caractérisé en ce que** le dispositif (11) pour la mise à disposition de la solution de dialyse présente une source d'eau (11a) et au moins une source de concentré (11b, 11c) et des moyens (M) pour mélanger l'eau et le(s) concentré(s), le dispositif pour modifier la propriété physique et/ou chimique de la solution de dialyse étant un dispositif modifiant le rapport de mélange eau - concentré(s).

5. Dispositif selon l'une quelconque des revendi-cations 1 à 4, **caractérisé en ce que** le disposi-tif de contrôle (35) présente un dispositif (36) pour définir le volume de solution de dialyse, qui s'écoule par un segment prescrit du parcours de la solution de dialyse (5), qui inclut le filtre stérile (15), entre le premier moment to, où la propriété physique et/ou chimique de la solution de dialyse est modifiée en amont du filtre stérile (15) avec le dispositif (11) pour modifier la propriété de la solution de dialyse, et le second moment t₁, où la modification de la propriété de la solution de dialyse est détectée avec le dispositif (28) pour mesurer la modifi-cation de la propriété de la solution de dialyse.

6. Dispositif selon la revendication 5, caracté-risé en ce que le dispositif de contrôle (35) présente des moyens (36) pour comparer le volume déterminé à au moins une valeur de référence prescrite.

7. Dispositif selon la revendication 5 ou 6, **caractérisé en ce que** sur le chemin de la solution de dialyse (5) est disposé un dispositif de bilan (18) pour faire le bilan de la solution de dialyse fraîche et de la solution de dialyse usée, qui présente au moins une chambre de bilan (17, 19) comprenant deux chambres partielles, dans laquelle une quantité prédéfinie de solution de dialyse est respectivement acheminée par cadences successives de la chambre de bilan, et les moyens (36) pour déterminer le volume de solu- tion de dialyse sont conçus de façon à ce que le volume de solution de dialyse puisse être déter-miné à partir du nombre de cadences de la chambre de bilan et de la quantité prédéfinie de solution de dialyse.

8. Dispositif selon l'une quelconque des revendi-cations 1 à 7, **caractérisé en ce que** le parcours de la solution de dialyse (5) présente un premier tronçon de conduite (9) d'une conduite d'alimen-tation de la solution de dialyse (6), lequel part du dispositif (11) de mise à disposition de la solution de dialyse et conduit à l'entrée de la première chambre (12) du filtre stérile (15), et un second tronçon de conduite (10) de la conduite d'alimentation de la solution de dialyse (6), lequel part de la seconde chambre (14) du filtre stérile (15) et conduit à l'entrée du dialyseur (1), et
■ le parcours de la solution de dialyse (5) pré-sente une conduite d'évacuation de la solution de dialyse (7), qui part de la sortie du dialyseur (1) et conduit à la sortie (16),
■ le dispositif (28) pour mesurer la propriété physique et/ou chimique du liquide dialyse étant un dispositif mesurant la propriété phy-sique et/ou chimique de la solution de dialyse dans la conduite d'évacuation de la solution de dialyse (7).

9. Dispositif selon la revendication 8, caracté-risé en ce que le parcours de la solution de dialyse (6) présente une conduite bypass (21) partant d'une sortie de la première chambre (12) du filtre stérile (15) et conduisant à la conduite d'évacuation de la solution de dialyse (7), le dispositif (28) pour mesurer la propriété physique et/ou chimique de la solution de dialyse étant un dispositif mesurant la propriété de la solution de dialyse dans la conduite d'évacuation de la solution de dialyse (7) en aval de la conduite bypass (21).

10. Méthode pour contrôler l'équipement d'un dis-positif de traitement extracorporel du sang, équipé d'un filtre stérile (15) à disposer sur le chemin de la solution de dialyse du dispositif de traitement du sang ou pour vérifier la taille correcte d'un filtre stérile (15) disposé sur le chemin de la solution de dialyse avant de commencer le traitement extracorporel du sang, avec les étapes suivantes :
- modification d'une propriété physique et/ou chimique de la solution de dialyse sur le chemin de la solution de dialyse en amont du filtre stérile (15) disposé ou à disposer sur le chemin de la solution de dialyse et mesure de la modification de la propriété de la solution de dialyse sur le chemin de la solution de dialyse en aval du filtre stérile disposé ou à disposer sur le chemin de la solution de dialyse,
- détermination de l'intervalle de temps Δt entre un premier moment t₀, où la propriété physique et/ou chimique de la solution de dialyse en amont du filtre stérile est modifiée, et un second moment t₁, où la modification de la propriété du liquide dialyse en aval du filtre stérile est détectée, et
- comparaison de l'intervalle de temps déterminé Δt ou d'une grandeur en corrélation avec celui-ci à au moins une valeur de référence prescrite.

11. Méthode selon la revendication 10, caracté-risée en ce que la propriété physique et/ou chimique de la solution de dialyse est la concentration d'une certaine substance dans la solution de dialyse.

12. Méthode selon la revendication 11, caracté-risée en ce que la conductibilité de la solution de dialyse est mesurée pour déterminer les concentrations d'une certaine substance dans la solution de dialyse.

13. Méthode selon l'une quelconque des revendi-cations 10 à 12, **caractérisée en ce que** pour la mise à disposition de la solution de dialyse, de l'eau est mélangée à au moins un concentré, le rapport de mélange eau - concentré(s) étant modifié pour modifier la propriété physique et/ou chimique de la solution de dialyse.

14. Méthode selon l'une quelconque des revendica-tions 10 à 13, **caractérisée en ce qu'**est déter-miné le volume de la solution de dialyse, qui s'écoule par un tronçon prescrit du parcours de la solution de dialyse, qui inclut le filtre stérile, entre le premier moment t₀, où la propriété physique et/ou chimique de la solution de dialyse en amont du filtre stérile est modifiée et le second moment t₁, où la modification de la propriété du liquide dialyse est détectée.

15. Méthode selon la revendication 14, caracté-risée en ce que le volume déterminé est comparé à au moins une valeur de référence prescrite.

16. Méthode selon l'une quelconque des revendi-cations 10 à 15, **caractérisée en ce que** le bilan est fait entre la solution de dialyse fraîche et la solution de dialyse usée dans un dispositif de bilan, qui achemine à des cadences successives de la chambre de bilan respectivement une quantité prédéfinie de solution de dialyse, le volume de solution de dialyse étant déterminé à partir du nombre de cadences de la chambre de bilan et de la quantité prédéfinie de solution de dialyse.
